# EUROPEAN PATENT SPECIFICATION

(11) **EP 2 476 798 B1**
(45) Date of publication and mention of the grant of the patent: **17.09.2014**
(21) Application number: 11151302.4
(22) Date of filing: 18.01.2011
(51) Int. Cl.: C12N 9/50, C09D 5/16, D06M 10/02, D06M 15/263, D06M 15/267, D06M 15/564, D06M 16/00

(54) **Antifouling textile materials comprising polymeric coatings and enzymes**
Fäulnisverhindernde Textilmaterialien mit polymeren Beschichtungen und Enzymen
Matériaux textiles antisalissures comprenant des revêtements polymères et des enzymes

(43) Date of publication of application: 18.07.2012
(73) Proprietor: Stazione Sperimentale per la Seta, 20133 Milano (IT); Cittadini S.p.A., 25050 Paderno Franciacorta, Brescia (IT)
(72) Inventor: Isella, Francesca, I-20045, Besana Brianza, MONZA E BRIANZA (IT); Donelli, Ilaria, I-20099, Sesto San Giovanni, MILANO (IT); Rosace, Giuseppe, I-24044, Dalmine, BERGAMO (IT); Alberti Fusi, Gabriella, I-22100, COMO (IT); Cittadini, Cesare, I-25050, Paderno Franciacorta, BRESCIA (IT); Freddi, Giuliano, I-20133, MILANO (IT)
(74) Representative: Long, Giorgio

(56) References cited:
- JP-A- 1 221 305
- DATABASE WPI Week 200121 Thomson Scientific, London, GB; AN 2001-205181 XP002649582, -& JP 2000 343073 A (TAKEUCHI Y) 12 December 2000 (2000-12-12)
- DATABASE WPI Week 198944 Thomson Scientific, London, GB; AN 1989-319081 XP002649583, -& JP 1 235579 A (NIPPON KAYAKU KK) 20 September 1989 (1989-09-20)
- DATABASE WPI Week 198413 Thomson Scientific, London, GB; AN 1984-077723 XP002649584, -& JP 59 028476 A (KANEBO LTD) 15 February 1984 (1984-02-15)

## Description

### Field of the invention

. The present invention relates to textile materials endowed with antifouling activity against organisms responsible for the biofouling of surfaces immersed in aquatic environments such as bacteria, protozoa, algae and invertebrates. More specifically, the object of the invention is a textile material in the form of yarn, net, woven, knitted and nonwoven fabrics that during its use has to remain submerged under sea water for variable lengths of time. Typical but not exclusive examples relevant for the invention are ropes, aquaculture nets, cover materials and other culture bags.

### Background art

. All surfaces immersed in aquatic environments are subjected to intense biofouling by various kinds of living organisms, including bacteria, protozoa, algae and invertebrates. Biofouling is initiated by attachment of a dispersal stage (i.e. spore or larva) of the organism, which secretes adhesive biopolymers (biofilm) to initiate settlement. This phase is vital for the completion of the life cycle of these organisms. In the marine environment barnacles are particularly pervasive foulers. The settlement stage of barnacles is a cypris larva (cyprid) that explores the surface using a method of reversible attachment known as temporary adhesion. This exploratory step occurs through secretion of glycoproteins from specialized glands. Once a suitable location has been selected, a second type of adhesive is secreted thus leading to permanent attachment and colonization of the surface.

. Aesthetic and functional damages to structures immersed in water may occur as a result of surface colonization. In the case of ship's hulls, for example, biofouling raises great economical and environmental issues because the increased surface roughness causes increased frictional drag, higher fuel consumption and higher emission levels of greenhouse gases.

. Antifouling strategies have long been based on the controlled release of biocides from paints and coatings applied onto the surface of immersed structures. The range of biocides comprises various metallic or organometallic compounds, most of which are toxic substances. Ideally, a biocide should target only the microorganism against which its use was intended. However, its release in the environment may adversely affect both plant and animal life. Thus, the continuous use of biocides for the control of biofouling inevitably raises concerns about effectiveness, resistance, biodegradability and environmental impact. A typical example is tributyltin (TBT), which has been extensively used until a global ban became effective from 2008 because its release and accumulation in the marine environment has been recognized to have adverse effects on marine life. Since then, the environmental impact of other potentially toxic biocides based on heavy metals, namely cupper, is under scrutiny.

. The ban of TBT and the restriction of use of other biocides based on heavy metals pose serious problems for the control of biofouling. A deeper understanding of the physical, chemical and biological phenomena underlying biofouling is needed to seek for novel non-toxic and environmentally friendly technologies able to prevent fouling. Studies have been oriented in various directions, such as: (i) imitation of the natural antifouling process, (ii) use of low energy surface approaches, (iii) weakening and degradation of the biofilm by enzymes.

. Marine organisms such as soft corals, sponges, echinoderms and seaweeds are considered a rich source of diverse antifouling substances because they are capable of remaining free from fouling while immersed in water by producing secondary metabolites which prevent the organisms from being fouled. Several antifouling compounds have been successfully identified and extracted, however compatibility of these compounds into commercial coatings with the desired release rates is still under investigation.

. Among the low surface energy materials tested the best are based on a silicone elastomer poly(dimethylsiloxane) (PDMS). These non-toxic technologies to control biofouling are also called "foul-release" silicone-based coatings in which a combination of low surface energy and low modulus reduces the adhesion strength of attached organisms.

. Another candidate antifouling technology is the incorporation of enzymes into coatings. The rationale behind this approach is to exploit the specificity and selectivity of enzymes for hydrolysing the biofilm deposited by the fouling organisms, thus preventing settlement, adhesion and colonization of surfaces immersed in the aquatic environment.

. Controlling fouling without the use of biocides essentially consists in impeding adhesion by reducing the strength and/or degrading the adhesive polymers as soon as they are secreted by the fouling organisms during settlement. An additional requisite is that enzymes attack adhesive polymers before they are cured by cross-linking, because after curing these polymers become less susceptible to enzyme degradation.

. Adhesive polymers secreted by fouling organisms are of various types. Most common are proteins, glycoproteins and polysaccharides. Different fouling organisms produce different adhesive polymers in widely different proportions. For example, the temporary adhesive secreted by cyprids is mainly composed of proteins. The extracellular polymeric matrix deposited by diatoms mostly consists of polysaccharides. Glycoproteins constitute the main component of the extracellular adhesives of the spores of algae. The variety of adhesive polymers secreted by fouling organisms implies that different hydrolytic enzymes are utilized for their degradation. Proteases, cellulases, amylases, xylanases and lipases are likely to display hydrolytic potential against biofilm components.

. Literature data show that serine proteases emerge as a group of enzymes with the greatest antifouling potential for a broad spectrum of fouling organisms being able to reduce the adhesion strength of spores, algae and cyprids (Aldred et al., The effects of a serine protease, Alcalase, on the adhesives of barnacle cyprids (Balanus amphitrite). Biofouling, 2008; 24(2) : 97-107; Olsen et al., Enzyme-based antifouling coatings: a review. Biofouling, 2007; 23(5): 369-383; Pettitt et al., Activity of commercial enzymes on settlement and adhesion of Cypris larvae of the barnacle Balanus amphitrite, spores of the green alga Ulva linza, and the diatom Navicula perminuta. Biofouling, 2004; 20(6): 299-311).

. Alcalase (EC 3.4.21.62; Novozymes, Denmark), a commercial preparation of the serine endopeptidase subtilisin, an enzyme initially obtained from *Bacillus subtilis,* reduces the adhesion of the green alga *U. linza* in a concentration-dependent manner and directly affects the ability of cyprids to attach to surfaces (Pettitt et al., 2004). In fact, it was seen that when barnacle cypris larvae explore a surface in the presence of the enzyme no footprints were observed and settlement was inhibited in a concentration-dependent manner (Aldred et al., 2008). A loss of Alcalase efficacy was observed with increasing the extent of biofilm curing (Pettitt et al., 2004).

. The concept of non toxic, environmentally friendly, enzyme-based antifouling treatments has recently been introduced by various authors who disclosed methods to integrate biocatalysts into paint and/or coating formulations with the aim of preventing or reducing the biofouling of structures occasionally or continuously immersed in water.

. WO 00/50521 published patent application describes an antifouling composition containing enzymes, microorganisms or both, which may also include epoxy resins and other additives such as inorganic salts in catalytic amount to enhance the activity of certain enzymes.

. WO 01/72911 A1 and US 2003/0166237 A1 disclose antifouling paint compositions comprising an enzyme, preferably the endopeptidase subtilisin (EC 3.4.21.62), and rosin, a natural resin obtained from conifers and some other plants. Biocides can be added to the paint formulation as antifouling enhancers. Other additives can be binders, fillers, pigments, solvents, plasticizers, etc.

. US 2005/0147579 published patent application relates to a coating composition containing an oxidase as biocatalyst able to act on a substrate, preferably produced by the same fouling organism, and to produce a peroxide, preferably hydrogen peroxide, as effective antifouling agent.

. WO 2006/002630 A1 published patent application discloses a self-polishing and antifouling coating composition comprising an enzyme with antifouling activity and a polymer hydrolysable in the aqueous environment or by the action of another specific biocatalyst able to use the said hydrolysable polymer as substrate.

. EP 1661955 A1 published patent application refers to an antifouling coating based on polymers, preferably of the acrylate type, carrying functional groups to which enzyme molecules can be covalently bonded.

. Enzymatic antimicrobial and antifouling coating and polymeric materials are disclosed in published patent application US 2009/0238811 A1.

. WO 2010/089598 A1 published patent application describes a process for preparing and using an antifouling composition containing cross-linked enzyme crystals or aggregates able to inhibit biofilm formation.

. The prior art previously cited mostly refers to coating and paint compositions suitable for application onto the surfaces of structures occasionally or continuously immersed in water, such as off-shore constructions, dock harbouring, ship's hulls, heat exchangers, water inlets, pilings, pipelines, marine markers and sensors, and so on. None of the prior art compositions and methods have been disclosed for the specific use on textile-based materials, such as ropes, culture bags and aquaculture nets.

. Differently from the above mentioned aquatic structures which are predominantly characterized by flat, rigid and, in some cases, very wide two-dimensional surfaces, textile-based structures usually display an extremely high surface-to-mass ratio and a complicated three-dimensional shape of the surfaces with fine details at the micrometer level. Moreover, textile materials are soft and ductile and tend to adapt their shape in response to external stresses, either during production (weaving, cutting, sewing, dyeing, finishing, etc.), handling, manufacturing, use and maintenance of textile-based devices.

. Antifouling coatings intended for textile materials have to fulfil several specific requirements in addition to those typical of coating and paint compositions suitable for other non textile aquatic structures. The coating components, especially the polymeric additives, must display a good compatibility with the different polymers which textile fibres are made of. The fluid composition must be able to penetrate into the micrometric spaces of the textile texture, thus forming a continuous film around individual fibres. The film must be very thin and soft in order not to impair handle properties of the textile material (softness, bend ability, etc.). Additionally, the film must possess a balanced combination of toughness and elasticity in order to resist to traction, bending and compression stresses without breaking.

. Based on the aforementioned requirements for antifouling textile materials, the present invention describes a process designed to integrate enzymes onto the surface of textile materials to endow the said textile material with an antifouling activity against fouling organisms present in aquatic environments.

JP 1221305 A discloses fishing nets coated with a resin bound to a protease, which resin needs a UV-light for cross-linking.

JP 2000343073 A discloses a method which is based on the immobilization of microorganisms directly onto the textile material.

JP 1235579 A describes fibres coated with a composition comprising a resin component and an enzyme, wherein said resin is photo-sensitive.

JP 59028476 A discloses a process including a step for treating the fibres with a solution comprising monofunctional vinyl or acrylic monomers, optionally a cross-linking agent and an enzyme. The polymer is formed in situ through plasma radicalization.

### Summary of the invention

. The present invention discloses a method for the preparation of an enzyme-based antifouling coating and for its application onto textile materials in an amount that is effective for preventing and/or reducing surface fouling without impairing the intrinsic properties of the fibres in terms of physical, chemical, mechanical and aesthetic properties. Typical but not exclusive examples of textile materials relevant for the invention are ropes, aquaculture nets, cover materials and other culture bags. The textile material carries a cross-linked polymeric coating with a biocatalysts immobilized into it, wherein the biocatalyst is an enzyme preferably belonging to the class of hydrolases. The degree of cross-linking is optimized to ensure durability of the coating, to maintain the catalytic activity of the enzyme for prolonged periods of time and to provide a constant availability of the bioactive agent at the water-material interface.

. Enzyme classes of interest for the invention comprise but are not limited to hydrolases, namely proteases, cellulases, amylases, xylanases and lipases.

. Enzymes can be used individually or in combination of two, three or more of them to ensure multifunctional performance against different biofilm components produced by fouling organisms.

. The antifouling coating formulation comprises aqueous solutions/dispersions of polymers able to entrap and stabilize enzymes and to form a continuous, elastic and durable film onto the surface of textile fibres.

. Preferred polymers include but are not limited to polyurethanes, acrylic omo- and copolymers, acetovinyl omo- and copolymers.

. Polyfunctional aziridine compounds are the cross-linking agents.

. Yarns, ropes and nets are the preferred textile supports, but also woven, knitted or nonwoven fabrics can be used to manufacture antifouling textile materials.

. These are preferably made of manmade fibres such as: polyester, polyamide, polyolefin, polyacrylonitrile, aramid, etc., and their blends.

. The surface of textile materials can be activated by means of chemical or physical systems, preferably but not exclusively by plasma treatment, in order to enhance adhesion of coating preparations.

. The antifouling textile material resulting from the process of the invention will be integrated into textile-based devices that have to remain temporarily or permanently immersed in aquatic environments.

. Further characteristics and the advantages of this invention will be better understood from the following detailed description of some embodiments thereof, which are provided by way of non-limiting examples.

### Brief description of the drawings

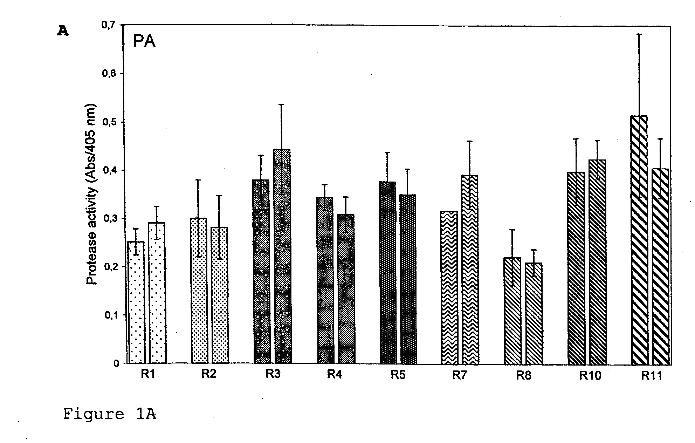

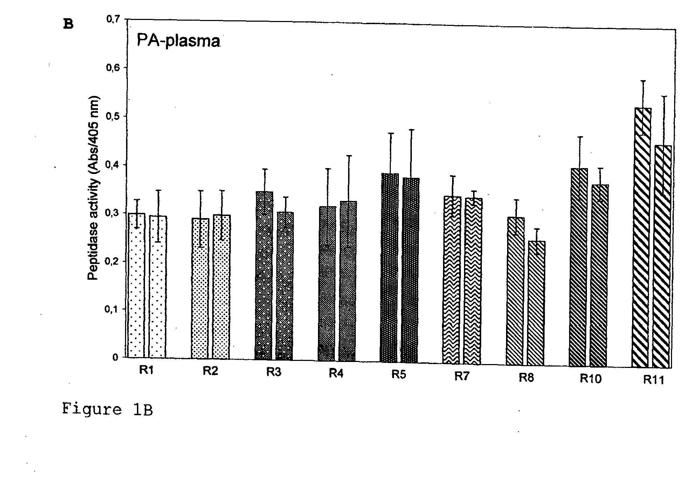

. Figures 1A and 1B: Activity of peptidase immobilized onto PA fabrics untreated (A) or pretreated with oxygen plasma (B). For sample captions consult Table I. Fabric samples were prepared and analysed in duplicate.

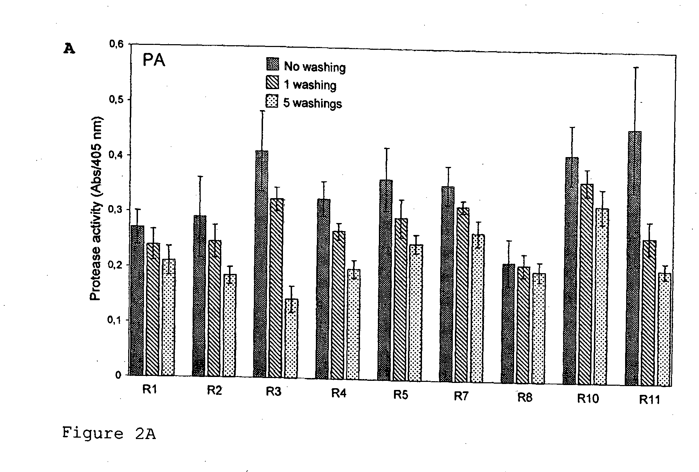

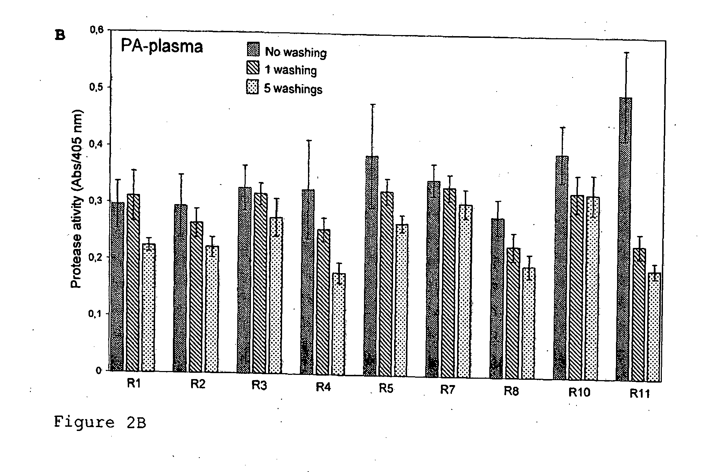

. Figures 2A and 2B: Activity of peptidase immobilized onto PA fabrics untreated (A) or pretreated with oxygen plasma (B) as a function of the number of washing cycles. For sample captions consult Table I. Fabric samples were prepared and analysed in duplicate.

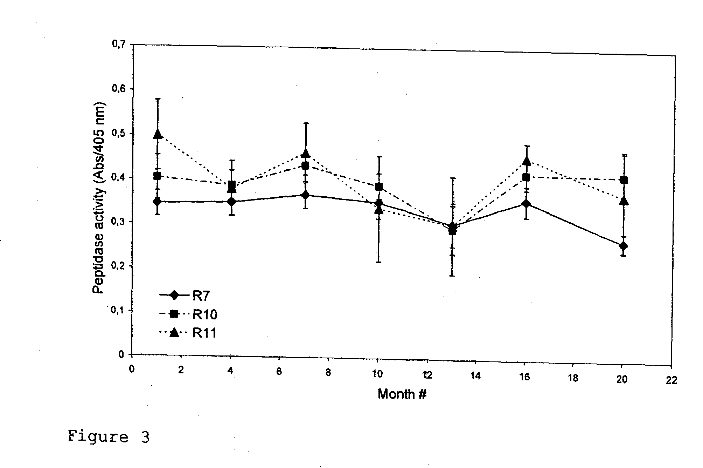

. Figure 3: Activity of peptidase immobilized onto PA fabrics (no plasma pretreatment) as a function of the storage time. For sample captions consult Table I. Fabric samples were prepared and analysed in duplicate.

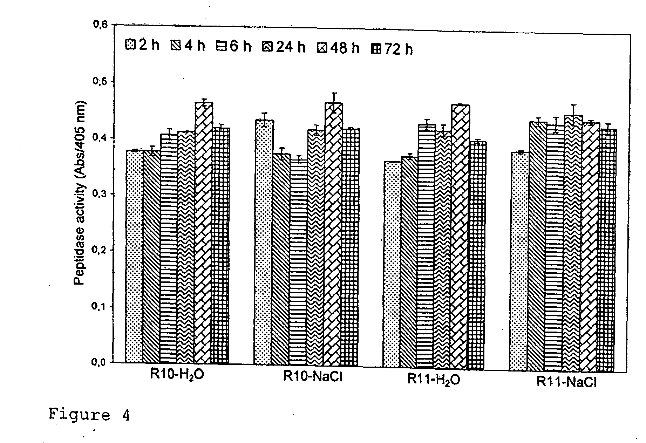

. Figure 4: Activity of peptidase immobilized onto PA fabrics (no plasma pretreatment) as a function of the time of immersion in pure water or simulated marine environment. NaCl concentration was 30 g/L. For sample captions consult Table I. Fabric samples were prepared and analysed in duplicate.

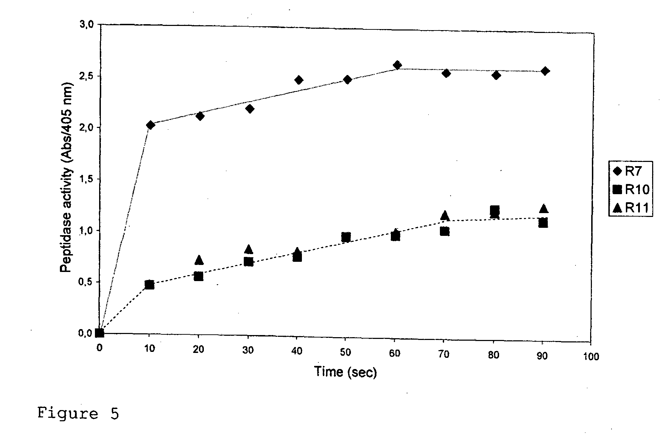

. Figure 5: Activity of peptidase immobilized onto PA nets (no plasma pretreatment).

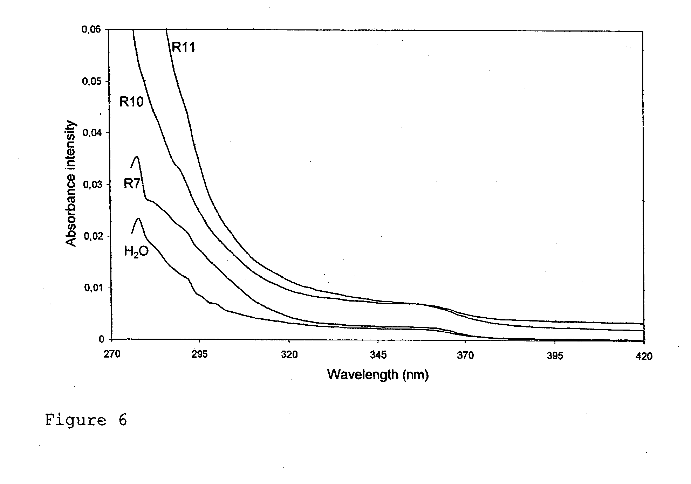

. Figure 6: Absorbance spectra of aqueous solutions after immersion for 24 hours of net samples coated with antifouling formulations comprising polymers R7, R10 and R11. The blank samples (H₂O) is pure distilled water.

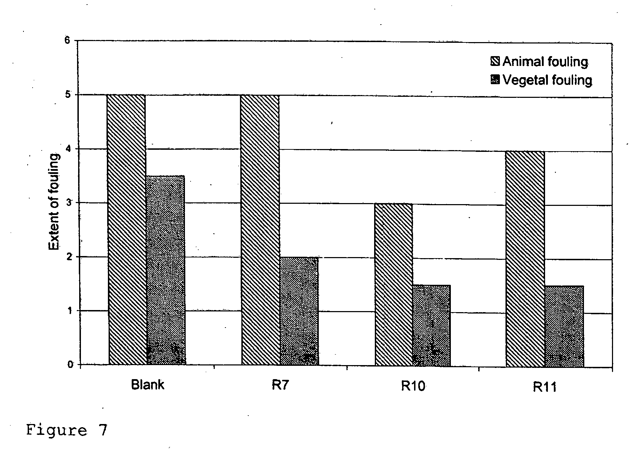

. Figure 7: Extent of plant and animal fouling determined on the front surface of PA fabric panels immersed in marine water for 70 days. Blank 1: uncoated PA fabric. Blank 2: PA fabric coated with polymer only.

### Detailed description of the invention

. The invention describes the preparation of bioactive textile materials comprising (i) a textile support in form of yarn, rope, net, woven, knitted, nonwoven fabric, possibly pre-treated with plasma; (ii) a bioactive coating endowed with antifouling activity consisting of one or more layers of a cross-linked polymer entrapping one or more enzymes.

. In an embodiment, the textile support is preliminarily activated by oxygen plasma treatment.

. In an embodiment, the textile support is coated with a polymeric layer loaded with enzyme, where the polymer component has a degree of cross-linking optimized to allow accessibility and availability of the enzyme at the water-material interface.

. In an embodiment, the textile support is coated with a polymeric layer loaded with enzyme, where the thickness of the polymer layer is adjusted to provide a soft, medium or hard handle to the textile material itself.

. In an embodiment, the textile support is coated with a polymeric layer loaded with enzyme, where the amount of enzyme is modulated to ensure the required level of antifouling activity.

. In an embodiment, the textile support is coated with a polymeric layer loaded with a mix of enzymes to ensure multifunctional performance against different biofilm components produced by fouling organisms.

. In an embodiment, the textile support is coated with multiple polymeric layers loaded with enzyme, where each layer may have different degree of cross-linking, or may be loaded with different amount of enzyme, or may contain different enzymes or enzyme mix.

. The steps leading to preparation of antifouling textile materials are as follows:
- Selection of the textile support and of the enzyme or enzyme mix according to end-use requirements,
- Optionally, plasma activation of the textile support on one or both surfaces,
- Preparation of the antifouling polymeric coating containing enzyme(s),
- Coating the optionally plasma activated textile support with the antifouling formulation,
- Optionally, repetition of the coating step for the deposition of multiple polymeric layers,
- Optionally, detection of enzyme(s) activity.

. The textile support can be a yarn, rope, net, woven, knitted or nonwoven fabric preferably made of manmade fibres (polyester, polyamide, polyolefin, polyacrylonitrile, aramid, etc.) or their blends.

. Composite textile supports comprising yarn reinforcements made of metal yarn, glass, carbon fibres or organic polymer materials such as aramids are also included.

. Other composite materials, polymer membranes and nanostructured matrices comprising nanofibres produced via the electrospinning technology are suitable supports for the process of the invention. The selection of the appropriate textile or textile composite support is driven by the targeted end-use application. Current knowledge of the structure/function/performance relationship of technical textiles is rapidly evolving under the pressure of market demand for functional, high-tech, engineered textile materials.

. The plasma treatment is a well known technology able to generate a wide range of active chemical species onto the surface of polymeric textile materials. These active chemical species, mostly radicals, can be exploited to enhance adhesion and binding strength of chemicals and polymers applied onto textiles by exhaust, impregnation or coating technologies. Vacuum plasma treatment under reduced atmosphere of oxygen gas has been used here to activate the textile support but also atmospheric plasma treatments in the presence of air or argon or other inert gases can be used for the sake of the process of the invention.

. Enzymes of interest for the invention are biocatalysts able to interact with target biopolymers forming the biofilms deposited by aquatic fouling organisms. Commercially available enzymes, endowed with high stability towards chemical and physical stresses, preferably but not exclusively belonging to the class of hydrolases, are suitable for the purpose of the invention. Examples of preferred enzymes include hydrolases acting on peptide bonds (peptidases, EC 3.4), on glucosidic linkages (glycolsylases, EC 3.2, including cellulase, amylase, xylanase, etc.), on ester bonds (EC 3.1, including esterase, lipase, cutinase, etc.), which are active against proteins, glycoproteins, polysaccharides, lipids and other biopolymers constituting the biofilm deposited by fouling organisms.

. All these enzymes can be used alone or in binary, ternary, etc. combination to widen the antifouling activity range. Commercial enzyme products are supplied in form of granules, powder or aqueous solution. Before use, the biocatalyst activity is tested by means of specific testing methods provided by the supplier or available from the scientific and technical literature. This allows determining the activity per unit weight or volume necessary to define the enzyme concentration in the application medium.

. The concentration of enzyme available within the coating formulation applied at the surface of the textile material is a key parameter for an effective antifouling performance. Based on the knowledge developed within this invention, enzyme concentration can vary between 1 and 100 g/L, preferably between 10 and 50 g/L, for a coating formulation that under standard impregnation conditions at room temperature, followed by squeezing between cylinders at 1-5 bar pressure, leave on the textile an amount of adsorbed liquor corresponding to 40-60% wet peak up.

. Any coating formulation containing enzymes must ensure the required stability of the biocatalyst under the conditions of manufacturing, storing, use and maintenance of the bioactive textile device produced thereof. As it will be easily understood from the detailed description of some embodiments of the invention, simply mixing the free enzyme with selected aqueous solutions/dispersions of polymers, followed by coating and thermal curing, is able to fulfil stability and activity requirements for prolonged times and under various physico-chemical stresses simulating use and maintenance conditions.

. Coating textile materials with polymers is a widely applied technology in the field of technical textiles. The coating process consists in the application of a viscous liquid, usually an aqueous solution or dispersion of a polymer, onto the surface of a textile material, followed by drying or curing processes. After evaporation of the solvent, usually water, a thin, continuous and solid film is formed whose morphological, chemical, physical and mechanical properties, as well as performance and durability are influenced by the kind of polymer and the coating technology used.

. Coating techniques depend on whether the textile material has to be coated on one or both sides. Impregnation techniques based on an impregnating trough followed by a passage through a couple of squeezing rollers are adequate for two sides coating. If coating has to be made only on one side or two different coatings have to be made on each side, other techniques must be used such as lick roll coating, knife coating, gravure coating, etc..

. Different polymers are used for coating technical textiles. Selection depends on required end-use performance in terms of gas permeability, transparency, abrasion, weathering, flame resistance, water and oil repellence, chemical and thermal stability, tensile strength, resilience, easy of processing, etc.

. Preferred polymers for the antifouling formulation include but are not limited to aqueous dispersions of polyurethanes, acrylic omo- and copolymers, acetovinyl omo- and copolymers with solid matter weight content of 30-60%, preferably with anionic and/or non-ionic character. Polymers must contain functional groups, preferably free carboxylic groups, with active hydrogen atoms able to react with polyfunctional aziridine compounds and to reticulate upon drying and thermal curing.

. The concentration of polymer in the antifouling formulation may vary in the range 10-400 g/L. Upon application on a textile material by coating technology, a formulation containing 200-400 g/L polymer will give strong, rigid and thick films, a formulation containing 100-200 g/L polymer will result in medium thickness and more elastic films, while a formulation containing 10-100 g/L polymer will give thin and soft films. The latter formulation can be used in particular, but not exclusively, for multilayer applications.

. Polyfunctional aziridine cross-linkers are versatile molecules with wide-ranging use in coatings and adhesive applications. These cross-linkers are used to improve both physical and chemical properties of polymer coatings. Due to their versatile reactivity, they allow reducing processing time while ensuring stability and durability of the coating. The aziridine end groups react with active hydrogen atoms, such as those found on carboxyl groups of acrylic or polyurethane resins.

. Trifunctional aziridine compounds, in particular pentaerythritol tris (3-(1-aziridinyl)propionate (C₂₀H₃₃O₇N₃, MW ≅ 430, CAS number 57116-45-7), are the preferred cross-linking agents.

. When all the three aziridine groups react with carboxyl groups of the polymer, a cross-linked network is formed. The extent of cross-linking and the cross-link density will depend on the amount of aziridine added to the formulated system and will influence the final properties of the coating film. Aziridine concentration of 0.001-0.05 mEq/g of cross-linkable polymer will give soft films able to swell in aqueous environments, thus leading to partial enzyme leaching from the coating at the water-material interface. A concentration of 0.05-0.5 mEq/g of cross-linkable polymer will impart strength, improve resistance and decrease the swelling ability of the film. At higher concentration, i.e. 0.5-5 mEq/g of cross-linkable polymer, the polyaziridine will result in highly cross-linked, stronger and less flexible films, with very limited swelling ability. Accordingly, the enzyme will be almost permanently entrapped into the polymer network.

. Preferred cross-linking conditions for the antifouling formulation of the invention containing the trifunctional aziridine compound are: drying at 80-100°C for 1-10 min, followed by curing at 20-50°C for 12-48 h. These conditions are safe for preserving the activity of the biocatalysts entrapped in the coating film.

. An important advantage of the method of the invention is that the whole process leading to the preparation of antifouling textile materials can be executed by using facilities available in textile mills, both for preparation or application of the coating formulation.

. Mixing of the components of the antifouling formulation can be made with the help of the dosing equipments currently used to prepare dyeing, printing and finishing recipes.

. Currently available textile technologies which bring fluids in close contact with the textile material, thus favouring adsorption, adhesion and physical or chemical anchoring of the active ingredients contained in the fluid onto the fibre surface, can be used for coating. These technologies include but are not limited to batch or continuous processes generally consisting in the impregnation of the textile material with a highly concentrated solution of the processing aids made by padding, coating or spraying, followed by fixation and drying steps.

. Last but not least, the method of the invention has the great advantage to provide a uniform coating of the textile material with a very thin film which does not impair handle properties (softness, bend ability, etc.) and processability (cutting, sewing, etc.) of the textile material coated with the antifouling formulation, while being at the same time tough and elastic enough to resist to traction, bending and compression stresses without breaking.

. Only for illustrative purposes, examples of preparation and testing of textile materials with antifouling activity are given here.

. EXAMPLE 1

### Coating a textile support with an antifouling composition and enzymatic activity assay

. A plain woven fabric made of 100% polyamide (PA) was used as textile support for coating with the antifouling composition. Samples of the size of a A4 sheet were cut from the fabric and used for the tests. The enzyme was a peptidase (subtilisin; EC 3.4.21.62) supplied by Novozymes A/S (Bagsvaerd, Denmark) under the commercial name Alcalase 2.5L. The polymers used, supplied by Forniture Tessili Riunite S.p.a. (Albano Sant'Alessandro, Bergamo, Italy), are listed in Table I.

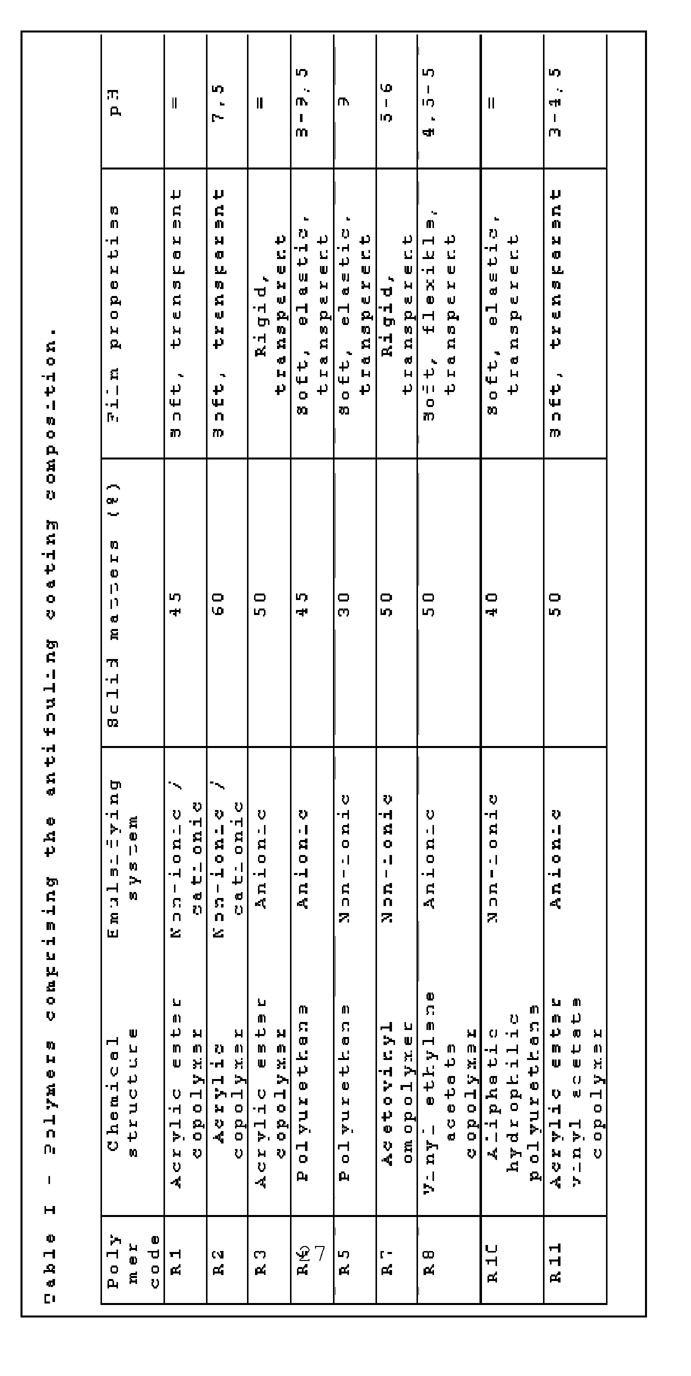

. In an embodiment, the PA fabric was treated with oxygen plasma under vacuum (50-100 Pa) with a Vacuum Plasma machine model KPR-180, equipped with RF generator operating at 10 KHz, current 200 A, fabric speed 10-15 m/min. Afterwards, the fabric was coated with the antifouling composition.

. In an embodiment, a 300 g/L aqueous polymer solution was mixed with trifunctional aziridine (0.1 mEq/g of cross-linkable polymer) at room temperature. Then, the peptidase solution was gently added to the previous solution so that the enzyme:polymer volume ratio was 1:5. After mixing, the solution was poured into the trough of a laboratory scale pad-drying machine. Samples of PA fabric were impregnated with a 2-roll padder Mathis Type HVF until a wet pick up of about 50 w% and then cured with a steamer unit Mathis Type DH. Curing temperature and time were 100°C and 2 min, respectively. Then, samples were left to polymerize at room temperature for 24 h before testing.

. The enzymatic activity of the peptidase immobilized onto the coated fabric was assayed for the ability to hydrolyze N-Succinyl-Ala-Ala-Pro-Phe p-Nitroanilide according to a modified published procedure (Sigma Enzymatic Assay). The formation of p-nitroaniline was detected spectrophotometrically at 405 nm.

. A 20 mM stock solution of the N-Succinyl-Ala-Ala-Pro-Phe p-Nitroanilide peptide was prepared in DMSO. Then, the test solution was prepared by adding 10 µl of the stock solution to 990 µl of 0.1 M potassium phosphate buffer at pH 8 containing 10 mM CaCl₂ and 0.005% Tween 80.

. Two strips of 1.5 x 8 cm were cut from a sample of fabric with immobilized peptidase and laid onto the surface of a testplate of a Varioskan Flash Multimode Reader (Thermo Scientific). Four drops, 13 µl each, of the test solution were pipetted onto the surface of one strip at regular distances. Four drops of water were pipetted onto the surface of the other strip (blank). The testplate was immediately inserted into the Varioskan reading unit and absorbance values at 405 nm were recorded for each drop of blank and test samples every 20 sec, over a total reading time of 5 min. The absorbance values recorded at 405 nm after 1 min incubation were used to express the activity of the immobilized peptidase.

. The activity of the peptidase immobilized onto PA fabrics using the different polymers listed in Table I is shown in Figures 1A and 1B. The preliminary plasma treatment resulted in more homogeneous coatings and lower variability of enzyme activity. The antifouling coating containing polymer R11 emerged as the best performing one in terms of enzyme activity level, suggesting a higher surface accessibility of enzyme molecules. The overall results confirm that the post-coating thermal treatment necessary to endow the polymeric film with good mechanical properties, stability and durability did not affect the integrity of the enzyme.

. EXAMPLE 2

### Stability and durability of the antifouling coating film under physical, chemical and mechanical stresses

. Samples of PA fabrics were prepared as described in EXAMPLE 1. In an embodiment, PA fabrics with immobilized peptidase were subjected to washing according to ISO 6330:2000 standard testing method to investigate the stability and durability of the bioactive film. The presence of Detergent ECE 77 (composition according to ISO 105-CO6) and of a centrifugation step simulated chemical and mechanical stresses, respectively. The peptidase activity was then assayed according to the test method reported in EXAMPLE 1 after 1 and 5 washings.

. The results reported in Figures 2A and 2B show that washing caused a decrease of peptidase activity, which was more evident after 5 washing cycles. This effect can be attributed either to leaching of enzyme from the film or to worsening of enzyme activity due to harsh chemical and mechanical conditions or to a concomitant effect of both factors. However, all samples retained a significantly high enzyme activity, confirming the good resistance of the antifouling polymeric film under adverse chemical/mechanical conditions.

. Interestingly, some samples displayed a proportionally lower decrease of peptidase activity, taking the activity of the respective non washed samples as a reference. Based on the level of peptidase activity retained after 5 washings, the performance of antifouling coatings can be ranked as follows: R8>R1>R10>R7 and R7>R3>R10>R1 for the PA fabric series without and with plasma pretreatment, respectively. These results highlight some effects of the preliminary physico-chemical surface treatment of the textile supports, which may affect the stability and durability of the antifouling coating, thus resulting in different levels of enzyme activity retention in response to chemical and mechanical stresses.

. EXAMPLE 3

### Shelf life stability of antifouling textile materials

. Samples of PA fabrics were prepared as described in EXAMPLE 1. In an embodiment, the antifouling textile materials were tested for peptidase activity soon after preparation, according to the assay method of EXAMPLE 1, and then were stored under normal ambient conditions for several months.

. The peptidase assay was repeated regularly over a period of 20 months. The results are shown in Figure 3. The peptidase activity remained at a significantly high level until month 20 for all the samples under examination. The minimum recorded at month 13 can be related to lower ambient temperature (winter season) which affected the enzyme activity. The long shelf life stability of the antifouling textile materials of the invention fits the time scale from processing of the textile material itself until manufacturing of the textile device (rope, net, etc.) to be used in the field.

. EXAMPLE 4

### Stability of the antifouling coating film under simulated sea water conditions

. Samples of PA fabrics were prepared as described in EXAMPLE 1. In an embodiment, the antifouling textile materials were immersed in pure water or in water containing 30 g/L of NaCl to simulate the average salinity of the marine environment. Specimens were taken at regular time intervals and assayed for peptidase activity according to the test method of EXAMPLE 1.

. Figure 4 illustrates that the saline aqueous environment did not affect the intensity of the enzyme response under the testing conditions of the peptidase assay.

. EXAMPLE 5

### Optimization of antifouling film thickness, assay of peptidase activity and evaluation of handle properties

. A series of samples of polyamide (PA) nets differing in weaving structure (with or without knot), yarn texture (titre) and colour (white or black) were selected for the antifouling treatment. Specimens with a size of 50 x 50 cm were cut from each net and used for the tests.

. In an embodiment, PA net samples of 50 x 50 cm were coated with antifouling formulations comprising: (i) polymers R7, or R10, or R11 at the concentration of 300 g/L, (ii) trifunctional aziridine as cross-linking agent at a concentration of 0.1 mEq/g of cross-linkable polymer, and (iii) peptidase (50 g/L). Impregnation was performed at room temperature until 40-60% wet pick up. Samples were dried at 100°C for 2 min and stored at room temperature for 24 h before testing. Samples were then subjected to peptidase activity assay to assess the rate and extent of bioactivity.

. Testing conditions were as follows: a specimens (10 mg) was cut from each net coated with the antifouling formulation and immersed in 4,5 ml of a solution of 20 mM N-Succinyl-Ala-Ala-Pro-Phe p-Nitroanilide prepared as described in Example 1. An aliquot of the solution was taken at regular intervals and the absorbance at 405 nm was measured spectrophotometrically. The reaction attained a plateau within 90 sec.

. As shown by the results of Figure 5, the peptidase activity was substantially high for all the samples examined, the one coated with the antifouling formulation comprising polymer R7 displaying the highest reaction rate and yield.

. In an embodiment, PA net samples of 50 x 50 cm were impregnated with three different antifouling formulations comprising polymers R7, R10, and R11. The composition was as follow: (i) formulation A: polymer 300 g/L, cross-linking agent 0.5 mEq/g of cross-linkable polymer, peptidase 50 g/L; (ii) formulation B: polymer 150 g/L, cross-linking agent 0.5 mEq/g of cross-linkable polymer, peptidase 10 g/L; (iii) formulation C: polymer 50 g/L, cross-linking agent 0.5 mEq/g of cross-linkable polymer, peptidase 2 g/L. Impregnation was performed at room temperature until 40-60% wet pick up. Samples were dried at 100°C for 2 min and stored at room temperature for 24 h before testing.

. Handle assessment was made by a panel of 3 experts who were asked to touch the different textile materials and to evaluate the main mechanical properties related to subjective handle evaluation like shear, bending, and compression behaviour. The results showed that with decreasing polymer concentration the handle became softer and softer: Formulation can be ranked as follows in terms of soft handle: A>B>C. The handle was also influenced by the net construction and texture. The kind of polymer used in the antifouling formulation also had a strong effect. Polymers can be ranked as follows in terms of softer handle imparted on nets: R11>R10>R8.

. EXAMPLE 6

### Leaching of antifouling coating components under aqueous environment

. Progressive detachment of thin superficial layers of the antifouling coating from the textile support once immersed in aqueous environment may play a key role in sustaining the antifouling performance through a kind of self-polishing activity which allows removing an outermost coating layer, together with all the fouling material possibly attached to it, and leads to exposition of new bioactive surfaces for a more prolonged antifouling effect.

. In an embodiment, PA net samples of 50 x 50 cm were coated with antifouling formulations as illustrated in EXAMPLE 5. Samples of 1 x 1 cm were cut and immersed in 10 ml distilled water and the absorbance spectra of the solution were measured at regular intervals. The results reported in Figure 6 show that the antifouling coatings differ in their leaching performance. The marked increase in absorbance in the 280-370 nm range indicates that polymer and enzyme components of the coating composition were released in the aqueous solution and that the leaching ability of a coating is influenced by the polymer used. In fact, R7 resulted more stable and released less material than R10, while R11 was more prone to release material into water.

. EXAMPLE 7

### Field tests in marine environment

. Samples of PA fabrics were prepared as described in EXAMPLE 1. In an embodiment, PA fabrics were coated with antifouling formulations comprising: (i) polymers R7, or R10, or R11 at the concentration of 300 g/L, (ii) trifunctional aziridine as cross-linking agent at 0.1 mEp/g of cross-linkable polymer, and (iii) peptidase (50 g/L). Impregnation was performed at room temperature until 40-60% wet pick up. Samples were dried at 100°C for 2 min and stored at room temperature for 24 h before testing.

. PA fabric samples coated with antifouling formulation, together with blank samples either uncoated or coated with polymer only, were mounted on frames and immersed in the marine water of Trieste harbour for 70 days, during the summer period (June-September). At regular intervals, at least once every 15 days, samples were taken out from water and examined to determine the amount of plant and animal fouling present on their front and back surface. The amount of fouling organisms adhering to the fabric surface was rated as follows: 1 = very very few; 2 = very few; 3 = few; 4 = few-medium; 5 = medium; 6 = medium-dense; 7 = dense.

. Figure 7 shows the results at 70 days of immersion in the marine water. Fabric samples coated with antifouling formulations comprising R7, R10 and R11 polymers and peptidase displayed better performance against plant fouling. Fabric samples coated with antifouling formulations comprising R10 and R11 polymers and peptidase displayed better performance against animal fouling. Blank samples, either uncoated or coated with polymer only, displayed lower performance, i.e. higher density of both animal and plant fouling.

## Claims

1. A process for obtaining a textile material endowed with antifouling properties, comprising the following steps:
- providing a textile support, a cross-linkable polymer, a cross-linker which is a polyfunctional aziridine and a hydrolase enzyme or enzyme mix,
- optionally, plasma activating said textile support on one or both surfaces,
- preparing an antifouling composition with the said cross-linkable polymer, the said cross-linker and the said hydrolase enzyme,
- coating the optionally plasma activated textile support with the said antifouling composition,
- optionally, repealing the coating step for the deposition of multiple polymeric layers,
- drying the said antifouling composition on the said textile support,
- cross-linking the said cross-linkable polymer with the said cross-linker.

2. The process of claim 1, wherein the said step of plasma activation is conducted by vacuum plasma treatment under reduced atmosphere of oxygen gas or by atmospheric plasma treatment in the presence of air or argon.

3. The process of claim 1 or 2, wherein the said step of providing a hydrolase enzyme comprises determining the enzymatic activity per unit weight or volume necessary to define the enzyme concentration in the application medium.

4. The process of any claim 1 to 3, wherein the said step of coating comprises coating both sides of the textile support by impregnation of the textile support at room temperature, followed by squeezing between cylinders at 1-5 bar pressure, to leave on the textile support an amount of adsorbed liquor corresponding to 40-60% wet pickup.

5. The process of any claim 1 to 4, wherein the said step of coating comprises coating a side of the textile support by lick roll coating, knife coating or gravure coating.

6. The process of any claim 1 to 5, wherein the said step of drying comprises drying the said antifouling composition at 80-100°C for 1-10 min.

7. The process of any claim 1 to 6, wherein the said step of cross-linking comprises curing the said cross-linkable polymer at 24-50°C for 12-48 h.

8. The process of any claim 1 to 7, wherein the said antifouling composition is an aqueous dispersion of the said cross-linkable polymer with the said cross-linker and the said hydrolase enzyme.

9. A textile material endowed with antifouling properties, as obtainable by a process according to any one of claims 1 to 8, comprising on at least one side of a textile support at least one layer of an antifouling composition containing a cross-linkable polymer cross-linked with a cross-linker which is a polyfunctional aziridine.

10. The textile material according to claim 9, wherein the said textile support is in form of yarn, rope, net, woven, knitted, nonwoven fabric.

11. The textile material according to claim 9 or 10, wherein the said textile support is pre-activated by plasma treatment with argon or inert gases or by oxygen plasma treatment.

12. The textile material according to any claim 9 to 11, wherein the said textile support is made of manmade fibres selected from polyester, polyamide, polyolefin, polyacrylonitrile, aramid or their blends or the said textile support comprises yarn reinforcements made of metal yarn, glass, carbon fibres or organic polymer materials such as aramids, or said textile support is a composite materials, polymer membrane or nanostructured matrix comprising nanofibres produced via the electrospinning technology.

13. The textile material according to any claim 9 to 12, wherein the said hydrolase enzyme is selected from peptidases acting on peptide bonds (EC 3.4), glycolsylases acting on glucosidic linkages (EC 3.2) including cellulase, amylase, xylanase, hydrolases acting on ester bonds (EC 3.1) including esterase, lipase, cutinase, which are active against proteins, glycoproteins, polysaccharides, lipids and other biopolymers constituting the biofilm deposited by fouling organisms.

14. The textile material according to any claim 9 to 13, wherein the said hydrolase enzyme in said antifouling composition has an enzyme concentration between 1 and 100 g/L, or between 10 and 50 g/L.

15. The textile material according to claim 14, wherein the said antifouling composition leaves on the textile support an amount of adsorbed liquor corresponding to 40-60% wet pickup.

16. The textile material according to claim 15, wherein the said cross-linkable polymer is selected from polyurethanes, acrylic homo- and copolymers, acetovinyl homo- and copolymers with solid matter weight content of 30-60%, preferably with anionic and/or non-ionic character.

17. The textile material according to any claim 9 to 16, wherein the said cross-linkable polymer has a concentration in the antifouling formulation in the range 10-400 g/L.

18. The textile material according to claim 17, wherein the said cross-linkable polymer has a concentration in the antifouling formulation in the range 200-400 g/L for obtaining strong, rigid and thick films; or in the range 100-200 g/L for obtaining medium thickness and elastic films; or in the range 10-100 g/L for obtaining thin and soft films.

19. The textile material according to claim 9, wherein the said polyfunctional aziridine cross-linker is pentaerythritol tris(3-(1-aziridinyl)propionate.

20. The textile material according to claim 19, wherein the said polyfunctional aziridine cross-linker is contained in the said antifouling composition in a concentration of 0.001-0.05 mEq/g of cross-linkable polymer to give soft firms able to swell in aqueous environments; or in a concentration of 0.05-0.5 mEq/g of cross-linkable polymer to give strength, improve resistance and decrease the swelling ability of the film; or in a weight concentration of 0.5-5 mEq/g of cross-linkable polymer to give highly cross-linked, stronger and less flexible films, with very limited swelling ability.

21. The textile material according to any claim 9 to 20, wherein the said textile is coated at both sides with more than one layer of an aqueous antifouling composition and wherein the said antifouling composition contains from 250 to 300 g/L of said cross-linkable polymer, from 0.001 to 5 mEq/g of said cross-linkable polymer or 0.05-1 mEq/g of said cross-linkable polymer of pentaerythritol tris(3-(1-aziridinyl)propionate, and from 50 to 100 g/L of a peptidase.

## Patentansprüche

1. Verfahren zum Erhalten eines Textilmaterials, welches mit Antifäulniseigenschaften ausgestattet ist, umfassend die folgenden Schritte:
- Bereitstellen eines Textilträgers, eines vernetzbaren Polymers, eines Vernetzers, welcher ein polyfunktionales Aziridin ist, und eines Hydrolaseenzyms oder einer Hydrolaseenzymmischung,
- gegebenenfalls, Plasmaaktivieren des Textilträgers auf einer oder beiden Oberflächen
- Zubereiten einer Antifäulniszusammensetzung mit dem vernetzbaren Polymer, dem Vernetzer und dem Hydrolaseenzym,
- Beschichten des gegebenenfalls plasmaaktivierten Textilträgers mit der Antifäulniszusammensetzung,
- gegebenenfalls, Wiederholen des Beschichtungsschritts zur Ablagerung mehrerer Polymerschichten,
- Trocknen der Antifäulniszusammensetzung auf dem Textilträger,
- Vernetzen des vernetzbaren Polymers mit dem Vernetzer.

2. Verfahren nach Anspruch 1, wobei der Schritt der Plasmaaktivierung durchgeführt wird durch Vakuum-Plasmabehandlung unter reduzierter Sauerstoffgasatmosphäre oder durch atmosphärische Plasmabehandlung in Gegenwart von Luft oder Argon.

3. Verfahren nach Anspruch 1 oder 2, wobei der Schritt des Bereitstellens eines Hydrolaseenzyms Bestimmen der enzymatischen Aktivität pro Gewichtseinheit oder Volumen umfasst, welche notwendig ist um die Enzymkonzentration in dem Applikationsmedium zu definieren.

4. Verfahren nach einem der Ansprüche 1 bis 3, wobei der Schritt des Beschichtens, Beschichten beider Seiten des Textilträgers durch Imprägnierung des Textilträgers bei Raumtemperatur umfasst, gefolgt durch Drücken zwischen Zylindern bei 1-5 bar Druck, um auf dem Textilträger eine Menge adsorbierter Feuchtigkeit zu lassen, welche 40-60% feuchte Aufnahme entspricht.

5. Verfahren nach einem der Ansprüche 1 bis 4, wobei der Schritt des Beschichtens, Beschichten einer Seite des Textilträgers durch Pflatschen (Lick-Roll Beschichten), Aufrakeln oder Tiefdruckbeschichten umfasst.

6. Verfahren nach einem der Ansprüche 1 bis 5, wobei der Schritt des Trocknens, Trocknen der Antifäulniszusammensetzung bei 80-100°C für 1-10 min umfasst.

7. Verfahren nach einem der Ansprüche 1 bis 6, wobei der Schritt des Vernetzens, Härten des vernetzbaren Polymers bei 20-50 °C für 12-48 h umfasst.

8. Verfahren nach einem der Ansprüche 1 bis 7, wobei die Antifäulniszusammensetzung eine wässrige Dispersion des vernetzbaren Polymers mit dem Vernetzer und dem Hydrolaseenzym ist.

9. Textilmaterial, welches mit Antifäulniseigenschaften ausgestattet ist, erhaltlich durch ein Verfahren nach einem der Ansprüche 1 bis 8, umfassend auf mindestens einer Seite eines Textilträgers mindestens eine Schicht einer Antifäulniszusammensetzung, welche ein vernetzbares Polymer, das mit einem Vernetzer, welcher ein polyfunktionales Aziridin ist, vernetzt ist enthält.

10. Textilmaterial nach Anspruch 9, wobei der Textilträger in Form von Garn, Leine, Netz, gewebtem Gewebe, gestricktem Gewebe, Vliesstoff ist.

11. Textilmaterial nach einem der Ansprüche 9 oder 10, wobei der Textilträger voraktiviert ist durch Plasmabehandlung mit Argon oder inerten Gasen oder durch Sauerstoffplasmabehandlung.

12. Textilmaterial nach einem der Ansprüche 9 bis 11, wobei der Textilträger aus künstlichen Fasern gemacht ist, ausgewählt aus Polyester, Polyamid, Polyolefin, Polyacrylnitril, Aramid oder deren Mischungen, oder der Textilträger Garnverstärkungen umfasst, welche aus Metallgarn, Glass, Kohlenstofffasern oder organischen Polymermaterialien, wie Aramide, gemacht sind, oder der Textilträger ein Composite-material, Polymermembran oder nanostrukturierte Matrix ist, umfassend durch die Elektrospinn-Technologie hergestellte Nanofasern.

13. Textilmaterial nach einem der Ansprüche 9 bis 12, wobei das Hydrolaseenzym ausgewählt ist aus Peptidasen, welche auf Peptidbindungen wirken (EC 3.4), Glykosylasen, welche auf glykosidische Verknüpfungen wirken (EC 3.2), welche Cellulase, Amylase, Xylanase beinhalten, Hydrolasen, welche auf Esterbindungen wirken (EC 3.1), welche Esterase, Lipase, Cutinase beinhalten, welche aktiv gegen Proteine, Glykoproteine, Polysaccharide, Lipide und andere Biopolymere sind, welche den durch Fäulnisorganismen abgelagerten Biofilm bilden.

14. Textilmaterial nach einem der Ansprüche 9 bis 13, wobei das Hydrolaseenzym in der Antifäulniszusammensetzung eine Enzymkonzentration zwischen 1 und 100 g/L, oder zwischen 10 und 50 g/L aufweist.

15. Textilmaterial nach Anspruch 14, wobei die Antifäulniszusammensetzung auf dem Textilträger eine Menge adsorbierter Feuchtigkeit lässt, welche 40-60% feuchte Aufnahme entspricht.

16. Textilmaterial nach Anspruch 15, wobei das vernetzbare Polymer ausgewählt wird aus Polyurethanen, acrylischen Homo- und Copolymeren, Acetovinyl Homo- und Copolymeren, mit einem Feststoffgehalt von 30-60 Gewichts-%, bevorzugt mit anionischem und/oder nicht-ionischem Charakter.

17. Textilmaterial nach einem der Ansprüche 9 bis 16, wobei das vernetzbare Polymer eine Konzentration in der Antifäulnisformulierung im Bereich 10-400 g/L aufweist.

18. Textilmaterial nach Anspruch 17, wobei das vernetzbare Polymer eine Konzentration in der Antifäulnisformulierung im Bereich 200-400 g/L aufweist zum Erhalten starker, rigider und dicker Filme; oder im Bereich 100-200 g/L zum Erhalten mitteldicker und elastischer Filme; oder im Bereich 10-100 g/L zum Erhalten dünner und weicher Filme.

19. Textilmaterial nach Anspruch 9, wobei der polyfunktionale Aziridin-Vernetzer Pentaerythritol tris(3-(1-aziridinyl)propionat) ist.

20. Textilmaterial nach Anspruch 19, wobei der polyfunktionale Aziridin-Vernetzer enthalten ist in der Antifäulniszusammensetzung in einer Konzentration von 0,001-0,05 mEq/g von vernetzbarem Polymer, um weiche Filme zu ergeben, welche in wässriger Umgebung quellen können; oder in einer Konzentration von 0,05-0,5 mEq/g von vernetzbarem Polymer, um Stärke zu ergeben, Wiederstandsfähigkeit zu verbessern und die Quellfähigkeit des Films zu verringern; oder in einer Gewichtskonzentration von 0,5-5 mEq/g von vernetzbarem Polymer, um hoch vernetzte, stärkere und weniger flexible Filme, mit sehr geringer Quellfähigkeit, zu ergeben.

21. Textilmaterial nach einem der Ansprüche 9 bis 20, wobei das Textil auf beiden Seiten mit mehr als einer Schicht einer wässrigen Antifäulniszusammensetzung beschichtet ist und wobei die Antifäulniszusammensetzung von 250 bis 300 g/L des vernetzbaren Polymers, von 0,001 bis 5 mEq/g des vernetzbaren Polymers oder 0,05-1 mEq/g des vernetzbaren Polymers von Pentaerythritol tris(3-(1-aziridinyl)propionat) und 50 bis 100 g/L einer Peptidase enthält.

## Revendications

1. Procédé pour obtenir un matériau textile doté de propriétés antisalissures, comprenant les étapes suivantes :
- fourniture d'un support textile, d'un polymère réticulable, d'un agent de réticulation qui est une aziridine polyfonctionnelle et d'une enzyme ou un mélange d'enzymes hydrolase(s),
- facultativement, activation par plasma dudit support textile sur l'une ou les deux surfaces,
- préparation d'une composition antisalissure avec ledit polymère réticulable, ledit agent de réticulation et ladite enzyme hydrolase,
- enduction du support textile facultativement activé par plasma avec ladite composition antisalissure,
- facultativement, répétition de l'étape d'enduction pour le dépôt de couches multiples de polymère,
- séchage de ladite composition antisalissure sur ledit support textile,
- réticulation dudit polymère réticulable avec ledit agent de réticulation.

2. Procédé de la revendication 1, dans lequel ladite étape d'activation par plasma est conduite par traitement par plasma sous vide sous une atmosphère réduite de gaz d'oxygène ou par traitement par plasma atmosphérique en présence d'air ou d'argon.

3. Procédé de la revendication 1 ou 2, dans lequel ladite étape de fourniture d'une enzyme hydrolase comprend la détermination de l'activité enzymatique par unité de poids ou de volume nécessaire pour définir la concentration d'enzyme dans le milieu d'application.

4. Procédé de l'une quelconque des revendications 1 à 3, dans lequel ladite étape d'enduction comprend l'enduction des deux côtés du support textile par imprégnation du support textile à température ambiante, suivie par l'essorage entre des cylindres à une pression de 1 à 5 bar, pour laisser sur le support textile une quantité de liqueur adsorbée correspondant à une absorption de 40 à 60 % d'humidité.

5. Procédé de l'une quelconque des revendications 1 à 4, dans lequel ladite étape d'enduction comprend l'enduction d'un côté du support textile par induction par rouleau lécheur, enduction à la lame ou enduction par gravure.

6. Procédé de l'une quelconque revendications 1 à 5, dans lequel ladite étape de séchage comprend le séchage de ladite composition antisalissure à 80 à 100 °C pendant 1 à 10 min.

7. Procédé de l'une quelconque des revendications 1 à 6, dans lequel ladite étape de réticulation comprend le durcissement dudit polymère réticulable à 20 à 50 °C pendant 12 à 48 h.

8. Procédé de l'une quelconque des revendications 1 à 7, dans lequel ladite composition antisalissure est une dispersion aqueuse dudit polymère réticulable avec ledit agent de réticulation et ladite enzyme hydrolase.

9. Matériau textile doté de propriétés antisalissures, tel que pouvant être obtenu par un procédé selon l'une quelconque des revendications 1 à 8, comprenant, sur au moins un côté d'un support textile, au moins une couche d'une composition antisalissure contenant un polymère réticulable réticulé avec un agent de réticulation qui est une aziridine polyfonctionnelle.

10. Matériau textile selon la revendication 9, dans lequel ledit support textile est sous forme de fil, corde, filet, textile tissé, tricoté, non-tissé.

11. Matériau textile selon la revendication 9 ou 10, dans lequel ledit support textile est pré-activé par traitement par plasma avec des gaz d'argon ou inertes ou par traitement par plasma à oxygène.

12. Matériau textile selon la revendication 9 à 11, dans lequel ledit support textile est constitué de fibres artificielles choisies parmi un polyester, un polyamide, une polyoléfine, un polyacrylonitrile, un aramide ou leurs mélanges ou bien ledit support textile comprend des renforcements de fil constitués de fil métallique, de verre, de fibres de carbone ou de matériaux polymères organiques tels que des aramides, ou bien ledit support textile est un matériau composite, une membrane de polymère ou une matrice nanostructurée comprenant des nanofibres produite par la technologie d'électrofilage.

13. Matériau textile selon la revendication 9 à 12, dans lequel ladite enzyme hydrolase est choisie parmi des peptidases agissant sur les liaisons peptidiques (EC 3.4), des glycosylases agissant sur les liaisons glucosidiques (EC 3.2) comprenant une cellulase, une amylase, une xylanase, des hydrolases agissant sur les liaisons ester (EC 3.1) comprenant une estérase, une lipase, une cutinase, qui sont actives contre les protéines, les glycoprotéines, les polysaccharides, les lipides et d'autres biopolymères constituant le biofilm déposé par les organismes contaminants.

14. Matériau textile selon l'une quelconque des revendications 9 à 13, dans lequel ladite enzyme hydrolase dans ladite composition antisalissure à une concentration d'enzymes comprises entre 1 et 100 g/l ou entre 10 et 50 g/l.

15. Matériau textile selon la revendication 14, dans lequel ladite composition antisalissure laisse sur le support textile une quantité de liqueur absorbée correspondant à une absorption de 40 à 60 % d'humidité.

16. Matériau textile selon la revendication 15, dans lequel ledit polymère réticulable est choisi parmi des polyuréthanes, des homo- et copolymères d'acrylique, des homo- et copolymères d'acétovinyle ayant une teneur en matières solides de 30 à 60 %, de préférence avec un caractère anionique et/ou non ionique.

17. Matériau textile selon l'une quelconque des revendications 9 à 16, dans lequel ledit polymère réticulable à une concentration dans la formulation antisalissure dans la plage de 10 à 400 g/l.

18. Matériau textile selon la revendication 17, dans lequel ledit polymère réticulable a une concentration dans la formulation antisalissure dans la plage de 200 à 400 g/l pour obtenir des films résistants, rigide et épais ; ou dans la plage de 100 à 200 g/l pour obtenir des films d'épaisseur moyenne et élastiques ; ou dans la plage de 10 à 100 g/l pour obtenir des films minces et souples.

19. Matériau textile selon la revendication 9, dans lequel ledit agent de réticulation d'aziridine polyfonctionnelle est le tris(3-(1-aziridinyl)propionate de pentaérythritol.

20. Matériau textile selon la revendication 19, dans lequel ledit agent de réticulation d'aziridine polyfonctionnelle est contenue dans ladite composition antisalissure à une concentration de 0,001 à 0,05 mEq/g de polymère réticulable pour obtenir des films souples pouvant gonfler dans des environnements aqueux ; ou à une concentration de 0,05 à 0,5 mEq/g de polymère réticulable pour conférer de la solidité, améliorer la résistance et diminuer la capacité de gonflement du film ; ou à une concentration en poids de 0,5 à 5 mEq/g de polymère réticulable pour obtenir des films hautement réticulés, plus solides et moins flexibles, avec une capacité de gonflement très limité.

21. Matériau textile selon l'une quelconque des revendications 9 à 20, dans lequel ledit textile est enduit sur les deux côtés avec plus d'une couche d'une composition antisalissure aqueuse et dans lequel ladite composition antisalissure contient de 250 à 300 g/l dudit polymère réticulable, de 0,001 à 5 mEq/g dudit polymère réticulable ou de 0,05 à 1 mEq/g dudit polymère réticulable de tris(3-(1-aziridinyl)propionate de pentaérythritol est de 50 à 100 g/l d'une peptidase.
